# EUROPEAN PATENT SPECIFICATION

(11) **EP 2 894 150 B1**
(45) Date of publication and mention of the grant of the patent: **17.05.2017**
(21) Application number: 13836036.7
(22) Date of filing: 14.03.2013
(51) Int. Cl.: C07D 231/14

(54) **METHOD FOR PRODUCING 1-SUBSTITUTED-3-FLUOROALKYLPYRAZOLE-4-CARBOXYLIC ACID ESTER**
VERFAHREN ZUR HERSTELLUNG 1-SUBSTITUIERTER 3-FLUORALKYLPYRAZOL-4-CARBONSÄUREESTER
PROCÉDÉ DE PRODUCTION D'UN ESTER D'ACIDE 3-FLUOROALKYLPYRAZOLE-4-CARBOXYLIQUE SUBSTITUÉ EN POSITION 1

(30) Priority: 05.09.2012 JP 2012194959
(43) Date of publication of application: 15.07.2015
(73) Proprietor: Tama Kagaku Kogyo Co., Ltd., Yashio-shi, Saitama 340-0807 (JP)
(72) Inventor: NOBESHIMA, Hirobumi, Yashio-shi Saitama 340-0807 (JP); KOYAMA, Naoki, Yashio-shi Saitama 340-0807 (JP); HARADA, Masayuki, Yashio-shi Saitama 340-0807 (JP)
(74) Representative: Blodig, Wolfgang
(86) International application number: PCT/JP2013/057275
(87) International publication number: WO 2014/038224

(56) References cited:
- WO-A1-2006/090778
- WO-A1-2010/009990
- WO-A1-2010/130532
- WO-A2-2008/059370
- WO-A2-2009/135808
- WO-A2-2011/012620

## Description

### Technical Field

The present invention relates to a method for producing a 1-substituted-3-fluoroalkylpyrazole-4-carboxylic acid ester that is useful as a synthetic intermediate or the like for medical and pharmaceutical products and agricultural chemicals.

### Background Art

When a 2-alkoxymethylene acyl acetic acid ester is reacted with a substituted hydrazine, a plurality of reaction sites are present in the 2-alkoxymethylene acyl acetic acid ester and therefore two kinds of pyrazole derivatives, a 1,3-disubstituted pyrazole-4-carboxylic acid ester and a 1,5-disubstituted pyrazole-4-carboxylic acid ester which are regioisomers, are generated. Therefore, in order to obtain an intended pyrazole derivative only, a purification step by silica gel column chromatography or the like that is industrially difficult to conduct becomes necessary.

As a related conventional technology, a method for producing a 1,3-dialkylpyrazole-4-carboxylic acid ester by reacting a 2-ethoxymethylene acyl acetic acid ester with an alkyl hydrazine in a solvent such as ethyl acetate has been proposed (Patent Literature 1). However, according to the production method described in Patent Literature 1, a mixture in which the 1,3-dialkylpyrazole-4-carboxylic acid ester (about 85%) and the 1,5-dialkylpyrazole-4-carboxylic acid ester (about 15%) are mixed together. Therefore, it has been necessary to conduct purification by distillation or the like in order to obtain the intended 1,3-dialkylpyrazole-4-carboxylic acid ester.

Moreover, a method for producing a 1-methyl-3-difluoromethylpyrazole-4-carboxylic acid ester by reacting ethyl 2-ethoxymethylene-4,4-difluoro-3-oxobutanoate with anhydrous methylhydrazine in the presence of a halogen-containing organic solvent such as a hydrofluorocarbon has been proposed (Patent Literature 2). However, even with the production method described in Patent Literature 2, a mixture containing a considerable amount of a regioisomer of the intended compound is obtained, and therefore there is still room for further improvement regarding an isomer ratio. Furthermore, since it is essential to use a special halogen-containing solvent in this production method, the production method has not necessarily been sufficient also in the aspect of versatility.

In order to improve the isomer ratio, a method for forming a pyrazole ring by reacting monomethylhydrazine with an aldehyde or ketone to make a hydrazone in advance and then reacting the hydrazone with ethyl 2-ethoxymethylene-4,4-difluoro-3-oxobutyrate has been proposed (Patent Literature 3). Moreover, a method of reacting methylhydrazine with ethyl 2-ethoxymethylene-4,4-difluoro acetoacetate in the presence of a base such as sodium hydroxide or potassium hydroxide in water or a mixed solvent of water and an organic solvent has been proposed (Patent Literature 4).

### Citation List

### Patent Literature

Patent Literature 1: Japanese Patent Laid-Open No. 2000-212166
Patent Literature 2: International Publication No. WO 2012/025469
Patent Literature 3: National Publication of International Patent Application No. 2011-519889
Patent Literature 4: Japanese Patent No. 4114754

### Summary of Invention

### Technical Problem

However, with the method described in Patent Literature 3, the aldehyde or ketone that is used in advance to obtain the hydrazone changes to a by-product, and the by-product is to be mixed with the pyrazole derivative that is a target substance. Therefore, since a step of conducting purification by separating the pyrazole derivative from the aldehyde or ketone becomes necessary, the method has not necessarily been a satisfiable one from the aspect of industrialization. Moreover, with the method described in Patent Literature 4, there is a problem that hydrolysis of the carboxylic acid ester as a target substance progresses to reduce yield. Furthermore, since the reaction is conducted in the presence of a base, there is also a problem that fluorine is liable to be detached, thus the concentration of fluorine in waste liquid increases, and thereby corrosion of a reaction apparatus progresses or waste liquid treatment becomes complicated.

The present invention has been made in consideration of such problems of the conventional technologies, and an object of the present invention is to provide a method for producing a 1-substituted-3-fluoroalkylpyrazole-4-carboxylic acid ester: by which method the intended regioisomer of the two regioisomers can be synthesized in high selectivity and high yield; which is highly versatile; and which is easily applicable to industrial process.

### Solution to Problem

The present inventors have made diligent studies to achieve the object to find out that the object can be achieved by making the following constitution, and have completed the present invention. Namely, according to the present invention, a method for producing a 1-substituted-3-fluoroalkylpyrazole-4-carboxylic acid ester is provided as shown below.
[1] A method for producing a 1-substituted-3-fluoroalkylpyrazole-4-caroxylic acid ester represented by the following general formula (3), the method comprising a step of adding, to a first reaction liquid containing an alkyl hydrazine represented by the following general formula (1) and a first organic solvent, a second reaction liquid containing an acyl acetic acid ester derivative represented by the following general formula (2) and a second organic solvent in 0.5 to 30 hours to react the first reaction liquid with the second reaction liquid at a reaction temperature of -5 to 80°C under stirring in the absence of a base and an acid, wherein the first organic solvent and the second organic solvent are each at least any one of benzene, toluene, xylene, chlorobenzene, dichlorobenzene, ethyl acetate, butyl acetate, and dimethyl carbonate, a total mass of the first organic solvent and the second organic solvent is 1 to 60 times a mass of the acyl acetic acid ester derivative, and an amount of the first organic solvent in a total amount of the first organic solvent and the second organic solvent is 40 to 95% by mass.

   R₁-NHNH₂ (1)

   (in the general formula (1), R₁ represents a C1-C6 alkyl group which may be substituted) 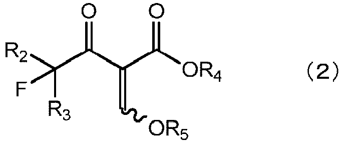 (in the general formula (2), R₂ represents a hydrogen atom or a halogen atom, R₃ represents a hydrogen atom, a fluorine atom, or a C1-C12 alkyl group which may be substituted with a chlorine atom or a fluorine atom, and R₄ and R₅ each independently represent a C1-C6 alkyl group) 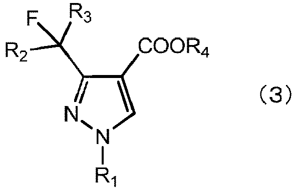 (in the general formula (3), R₁ represents a C1-C6 alkyl group which may be substituted, R₂ represents a hydrogen atom or a halogen atom, R₃ represents a hydrogen atom, a fluorine atom, or a C1-C12 alkyl group which may be substituted with a chlorine atom or a fluorine atom, and R₄ represents a C1-C6 alkyl group)
[2] The method for producing a 1-substituted-3-fluoroalkylpyrazole-4-carboxylic acid ester according to [1], wherein the amount of the first organic solvent in the total amount of the first organic solvent and the second organic solvent is 65 to 92% by mass.
[3] The method for producing a 1-substituted-3-fluoroalkylpyrazole-4-carboxylic acid ester according to [1] or [2], wherein the first organic solvent and the second organic solvent are each at least any one of toluene, xylene, and ethyl acetate.
[4] The method for producing a 1-substituted-3-fluoroalkylpyrazole-4-carboxylic acid ester according to any one of [1] to [3], wherein the total mass of the first organic solvent and the second organic solvent is 5 to 60 times the mass of the acyl acetic acid ester derivative.
[5] The method for producing a 1-substituted-3-fluoroalkylpyrazole-4-carboxylic acid ester according to any one of [1] to [4], wherein the amount of the acyl acetic acid ester derivative contained in the second reaction liquid is 0.8 to 1.2 molar equivalents relative to the amount of the alkyl hydrazine.

### Advantageous Effects of Invention

According to the method for producing a 1-substituted-3-fluoroalkylpyrazole-4-carboxylic acid ester of the present invention, the intended regioisomer of the two regioisomers can be synthesized in high selectivity and high yield. Moreover, the method for producing a 1-substituted-3-fluoroalkylpyrazole-4-carboxylic acid ester of the present invention is highly versatile and is easily applicable to industrial process.

### Brief Description of Drawings

[Figure 1] Figure 1 is a high performance liquid chromatography (HPLC) chart for a white crystal obtained by Example 1.
[Figure 2] Figure 2 is a high performance liquid chromatography (HPLC) chart for a yellow-orange crystal obtained by Comparative Example 1.

### Description of Embodiments

Hereinafter, the embodiments of the present invention will be described, however the present invention is not limited to the following embodiments. The present invention is a method for producing a 1-substituted-3-fluoroalkylpyrazole-4-carboxylic acid ester represented by the following general formula (3) (hereinafter, simply referred to also as "production method of the present invention"). 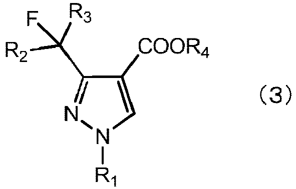 (in the general formula (3), R₁ represents a C1-C6 alkyl group which may be substituted, R₂ represents a hydrogen atom or a halogen atom, R₃ represents a hydrogen atom, a fluorine atom, or a C1-C12 alkyl group which may be substituted with a chlorine atom or a fluorine atom, and R₄ represents a C1-C6 alkyl group)

The production method of the present invention comprises a step (hereinafter, also referred to as "reaction step") of adding, to a first reaction liquid containing an alkyl hydrazine represented by the following general formula (1) and a first organic solvent, a second reaction liquid containing an acyl acetic acid ester derivative represented by the following general formula (2) and a second organic solvent to react the first reaction liquid with the second reaction liquid under stirring in the absence of a base and an acid.

**R₁-NHNH₂** **(1)**

(in the general formula (1), R₁ represents a C1-C6 alkyl group which may be substituted) 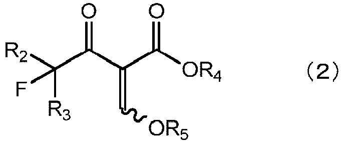 (in the general formula (2), R₂ represents a hydrogen atom or a halogen atom, R₃ represents a hydrogen atom, a fluorine atom, or a C1-C12 alkyl group which may be substituted with a chlorine atom or a fluorine atom, and R₄ and R₅ each independently represent a C1-C6 alkyl group)

Specific examples of the C1-C6 alkyl group represented by R₁ in the general formulas (1) and (3) include a methyl group, an ethyl group, a propyl group, a cyclopropylmethyl group, a butyl group, an isobutyl group, a pentyl group, a hexyl group, and so on. These alkyl groups may be substituted with a halogen atom or the like. Specific examples of the C1-C6 alkyl group which may be substituted include a 2-chloroethyl group, a 2-bromoethyl group, a 2-hydroxyethyl group, a 2,2,2-trifluoroethyl group, a 3-chloropropyl group, and so on.

As an alkyl hydrazine represented by the general formula (1), a generally available alkyl hydrazine may be used as it is or an alkyl hydrazine that is produced by a publicly known method may be used. Moreover, with regard to these alkyl hydrazines, any of an anhydride, a hydrated compound, and an aqueous solution can be used.

Specific examples of the halogen atom represented by R₂ in the general formulas (2) and (3) include a fluorine atom, a chlorine atom, a bromine atom, and so on.

Specific examples of the C1-C12 alkyl group which may be substituted with a chlorine atom or a fluorine atom, the C1-C12 alkyl group represented by R3 in the general formulas (2) and (3) include a trifluoromethyl group, a difluoromethyl group, a chloro-difluoromethyl group, a pentafluoroethyl group, a perfluoropropyl group, a perfluoropentyl group, a 1,1,2,2,3,3,4,4,5,5-decafluoropentyl group, a perfluorohexyl group, a perfluorononyl group, a perfluorodecyl group, a perfluorododecyl group, and so on.

Specific examples of the C1-C6 alkyl group represented by R₄ and R₅ in the general formula (2) each include a methyl group, an ethyl group, a propyl group, an isopropyl group, a butyl group, an isobutyl group, a pentyl group, a hexyl group, and so on. Moreover, specific examples of the C1-C6 alkyl group represented by R₄ in the general formula (3) include a methyl group, an ethyl group, a propyl group, an isopropyl group, a butyl group, an isobutyl group, a pentyl group, a hexyl group, and so on.

As an acyl acetic acid ester derivative represented by the general formula (2), a commercially available acyl acetic acid ester derivative may be used as it is or an acyl acetic acid ester derivative that is produced in accordance with usual techniques of organic synthesis may be used. For example, the acyl acetic acid ester derivative represented by the general formula (2) can easily be produced by reacting a β-keto carboxylic acid ester, the β-keto carboxylic acid ester obtained by a Claisen condensation reaction of a fluorine-containing carboxylic acid ester and an acetic acid ester, with an ortho-formic acid ester in the presence of acetic anhydride.

In the reaction step of the production method of the present invention, the second reaction liquid is added to the first reaction liquid by, for example, a dropping method or the like to react the alkyl hydrazine contained in the first reaction liquid with the acyl acetic acid ester derivative contained in the second reaction liquid. The first organic solvent is contained in the first reaction liquid together with the alkyl hydrazine represented by the general formula (1). As a first organic solvent, at least any one of an aromatic hydrocarbon solvent and an ester solvent can be used for example. Specific examples of the aromatic hydrocarbon solvent include benzene, toluene, xylene, chlorobenzene, dichlorobenzene, and so on. Moreover, specific examples of the ester solvent include ethyl acetate, butyl acetate, dimethyl carbonate, and so on. Among these organic solvents, toluene, xylene, and ethyl acetate are preferable.

The second organic solvent is contained in the second reaction liquid together with the acyl acetic acid ester derivative represented by the general formula (2). Specific examples of the second organic solvent include the same organic solvent as the first organic solvent including preferable ones. In addition, the kinds of the first organic solvent and the second organic solvent may be the same or different.

In the reaction step of the production method of the present invention, the reaction is allowed to progress by adding the second reaction liquid to the first reaction liquid and stirring the resultant mixture in the absence of a base and an acid. By reacting the alkyl hydrazine with the acyl acetic acid ester derivative in the absence of a base and an acid, hydrolysis of the generated 1-substituted-3-fluoroalkylpyrazole-4-carboxylic acid ester can effectively be suppressed. Therefore, the 1-substituted-3-fluoroalkylpyrazole-4-carboxylic acid ester that is a target substance can be obtained in high yield. Furthermore, by adding the second reaction liquid to the first reaction liquid, namely by allowing the reaction of both compounds to progress under the condition that an excessive amount of the alkyl hydrazine exists relative to the amount of the acyl acetic acid ester derivative, a ratio (reaction selectivity) of generating the target compound represented by the following general formula (3) can be enhanced. 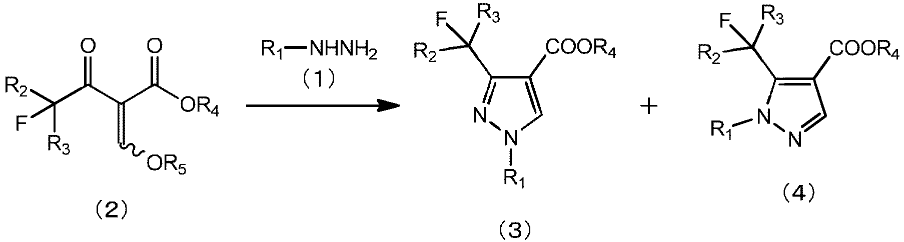 (in the general formulas (1), (3), and (4), R₁ represents a C1-C6 alkyl group which may be substituted. In the general formulas (2), (3), and (4), R₂ represents a hydrogen atom or a halogen atom, R₃ represents a hydrogen atom, a fluorine atom, or a C1-C12 alkyl group which may be substituted with a chlorine atom or a fluorine atom, and R₄ represents a C1-C6 alkyl group. Moreover, in the general formula (2), R₅ represents a C1-C6 alkyl group.)

Moreover, the total mass of the first organic solvent and the second organic solvent (the total mass of the organic solvents) is set to 1 to 60 times, preferably 5 to 50 times, and more preferably 6 to 40 times the mass of the acyl acetic acid ester derivative. Namely, the reaction selectivity can be enhanced by reacting the acyl acetic acid ester derivative with the alkyl hydrazine in a state that the acyl acetic acid ester derivative is appropriately diluted with an organic solvent.

Furthermore, the amount of the first organic solvent in the total mass of the first organic solvent and the second organic solvent (the total amount of the organic solvents) is set to 40 to 95% by mass, preferably 65 to 92% by mass, and more preferably 67 to 90% by mass. Namely, by allowing the alkyl hydrazine contained in the first organic solvent to make contact with the acyl acetic acid ester derivative contained in the second organic solvent to react in a state that the respective compounds are appropriately diluted, the reaction selectivity can be enhanced. As described here, by suitably controlling the amount of organic solvents to be used, the 1-substituted-3-fluoroalkylpyrazole-4-carboxylic acid ester that is an intended regioisomer of the two regioisomers can be generated in high selectivity without reacting the alkyl hydrazine with the acyl acetic acid ester derivative in the presence of a base.

In addition, in the production method of the present invention, the second reaction liquid is added to the first reaction liquid not at a time but slowly over appropriate time by a dropping method or the like. Thereby, it becomes possible to generate the intended 1-substituted-3-fluoroalkylpyrazole-4-carboxylic acid ester in higher selectivity. Specifically, the second reaction liquid containing an acyl acetic acid ester derivative is added to the first reaction period in 0.5 to 30 hours, preferably 1 to 25 hours. When the time taken for addition is less than 0.5 hours, the reaction selectivity is lowered. On the other hand, the time taken for addition may exceed 30 hours, however the reaction selectivity enhancement effect tends to hit a peak when the time taken for addition exceeds 30 hours. In addition, the amount of the acyl acetic acid ester derivative contained in the second reaction liquid is usually 0.8 to 1.2 molar equivalents, preferably 0.85 to 1.15 molar equivalents relative to the amount of the alkyl hydrazine in the first reaction liquid.

It is preferable that the reaction temperature in the reaction step is set to -5 to 80°C, more preferably 0 to 60°C. When the reaction temperature is lower than -5°C, the reaction tends to be hard to progress. On the other hand, when the reaction temperature exceeds 80°C, the reaction selectivity tends to be lowered. The yield and the reaction selectivity can further be improved by controlling the reaction temperature in the above-described range.

According to the above-described reaction step, the regioisomer (target compound) represented by the general formula (3) of the two regionisomers represented by the general formula (3) and the general formula (4)respectively can be generated in high selectivity and high yield. Therefore, when the extraction operation or the like is conducted after the reaction step in accordance with usual techniques of organic synthesis, the target compound having high purity can be obtained. In addition, when the target compound having higher purity is required, recrystallization, washing, distillation, or the like may be conducted as necessary.

### Examples

Hereinafter, the present invention will be described specifically based on Examples, however the present invention is not limited to these Examples. In addition, "parts" and "%" in Examples and Comparative Examples are based on mass unless otherwise noted.

### (Example 1)

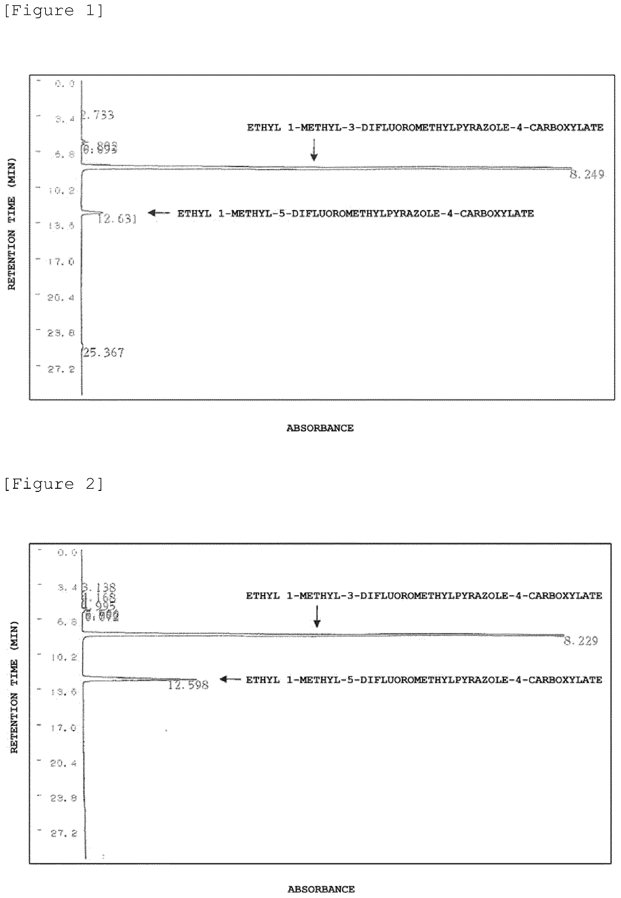

In a 100 ml four-necked flask equipped with a thermometer and a stirrer, 49.55 g of toluene and 15.92 g (0.047 mol) of a 13.5% monomethylhydrazine aqueous solution were placed, and stirring was started. Into the resultant mixture, a mixed solution of 8.88 g (0.040 mol) of ethyl 2-ethoxymethylene-4,4-difluoroacetoacetate represented by the following formula (2-1) and 9.95 g of toluene was dropped in 16 hours using a metering pump at an internal temperature of 5°C. After the completion of dropping, stirring was conducted at an internal temperature of 5°C for further 1 hour. A toluene layer obtained by separating the toluene layer from an aqueous layer was evaporated to dryness under reduced pressure to obtain 7.98 g (yield 92.8%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC), and quantitative analysis was conducted by an absolute calibration curve method to find that a generation ratio (isomer ratio) of the former compound to the latter compound was 97.4:2.6 (HPLC area ratio). In addition, an HPLC chart is shown in Figure 1. Moreover, the HPLC conditions are shown below.
- Column: product name "Inertsil ODS-3" (4.6 ×150 mm, manufactured by GL Sciences Inc.)
- Temperature: 40°C
- Flow rate: 1.0 mL/min
- Fluid phase: liquid A; acetonitrile, liquid B; 0.2% by volume of acetic acid aqueous solution, A:B = 45:55
- Detector (wave length): 220 nm

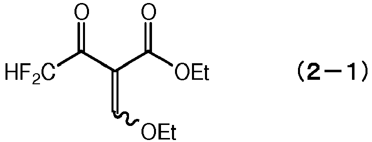

In an eggplant-shaped flask having a 30 mL side tube, 7.00 g of the obtained white crystal was charged, then 10 g of heptane and 1.7 g of acetone were added thereto, and thereafter the temperature was raised to 70°C under stirring with a magnetic stirrer to dissolve the white crystal. When warming was stopped and the resultant mixture was slowly cooled by air to 25°C, a white crystal was precipitated. The precipitated white crystal was filtered under reduced pressure and thereafter dried under reduced pressure to obtain a 5.99 g white crystal of ethyl 1-methyl-3-difluoromethylpyrazole-4-carboxylate. The analysis result of the obtained white crystal by ¹H-NMR is shown below.
¹H-NMR (CDCl₃, TMS, ppm) : δ1.35 (t, J = 7.2 Hz, 3H), 3.96 (s, 3H), 4.31 (q, J = 7.2, 2H), 7.11 (t, J=54, 1H), and 7.90 (s, 1H)

### (Example 2)

In a 50 ml four-necked flask equipped with a thermometer and a stirrer, 4.45 g of toluene and 12.0 g (0.022 mol) of an 8.8% monomethylhydrazine aqueous solution were placed, and stirring was started. Into the resultant mixture, 8.90 g (0.02 mol) of a 50% toluene solution of ethyl 2-ethoxymetylene-4,4-difluoroacetoacetate was dropped in 4 hours using a metering pump at an internal temperature of 5°C. After the completion of dropping, an aqueous layer and a toluene layer were separated. The obtained toluene layer was evaporated to dryness under reduced pressure to obtain 3.90 g (yield 95.5%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC) to find that a generation ratio (isomer ratio) of the former compound to the latter compound was 94.1:5.9.

### (Example 3)

In a 50 ml four-necked flask equipped with a thermometer and a stirrer, 8.60 g of toluene and 12.0 g (0.022 mol) of an 8.8% monomethylhydrazine aqueous solution were placed, and stirring was started. Into the resultant mixture, 13.1 g (0.02 mol) of a 34% toluene solution of ethyl 2-ethoxymetylene-4,4-difluoroacetoacetate was dropped in 4 hours using a metering pump at an internal temperature of 5°C. After the completion of dropping, an aqueous layer and a toluene layer were separated. The obtained toluene layer was evaporated to dryness under reduced pressure to obtain 3.95 g (yield 96.7%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC) to find that a generation ratio (isomer ratio) of the former compound to the latter compound was 94.1:5.9.

### (Example 4)

In a 50 ml four-necked flask equipped with a thermometer and a stirrer, 18.00 g of toluene and 8.00 g (0.022 mol) of a 13.1% monomethylhydrazine aqueous solution were placed, and stirring was started. Into the resultant mixture, 13.10 g (0.02 mol) of a 34% toluene solution of ethyl 2-ethoxymetylene-4,4-difluoroacetoacetate was dropped in 24 hours using a metering pump at an internal temperature of 5°C. After the completion of dropping, an aqueous layer and a toluene layer were separated. The obtained toluene layer was evaporated to dryness under reduced pressure to obtain 4.00 g (yield 98.0%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC) to find that a generation ratio (isomer ratio) of the former compound to the latter compound was 98.9:1.1.

### (Example 5)

In a 100 ml four-necked flask equipped with a thermometer and a stirrer, 24.8 g of toluene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 4.92 g of toluene was dropped in 0.5 hours using a metering pump at an internal temperature of 5°C. After the completion of dropping, an aqueous layer and a toluene layer were separated. The obtained toluene layer was evaporated to dryness under reduced pressure to obtain 3.68 g (yield 90.0%) of a white crystal comprising ethyl 1-methyl-3-difluoromethylpyrazole-4-carboxylate and ethyl 1-methyl-5-difluoromethylpyrazole-4-carboxylate. The obtained yellow oil was analyzed by high performance liquid chromatography (HPLC) to find that a generation ratio (isomer ratio) of the former compound to the latter compound was 91.5:8.5.

### (Example 6)

In a 100 ml four-necked flask equipped with a thermometer and a stirrer, 24.8 g of toluene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 4.97 g of toluene was dropped in 22 hours using a metering pump at an internal temperature of 50°C. After the completion of dropping, an aqueous layer and a toluene layer were separated. The obtained toluene layer was evaporated to dryness under reduced pressure to obtain 3.86 g (yield 94. 5%) of yellow oil comprising ethyl 1-methyl-3-difluoromethylpyrazole-4-carboxylate and ethyl 1-methyl-5-difluoromethylpyrazole-4-carboxylate. The obtained yellow oil was analyzed by high performance liquid chromatography (HPLC) to find that a generation ratio (isomer ratio) of the former compound to the latter compound was 94.2:5.8.

### (Example 7)

In a 100 ml four-necked flask equipped with a thermometer and a stirrer, 24.8 g of toluene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 4.97 g of toluene was dropped in 1 hour using a metering pump at an internal temperature of 5°C. After the completion of dropping, an aqueous layer and a toluene layer were separated. The obtained toluene layer was evaporated to dryness under reduced pressure to obtain 3.86 g (yield 94.5%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC) to find that a generation ratio (isomer ratio) of the former compound to the latter compound was 97.6:2.4.

### (Example 8)

In a 200 ml four-necked flask equipped with a thermometer and a stirrer, 79.92 g of toluene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 8.88 g of toluene was dropped in 18 hours using a metering pump at an internal temperature of 5°C. After the completion of dropping, an aqueous layer and a toluene layer were separated. The obtained toluene layer was evaporated to dryness under reduced pressure to obtain 4.00 g (yield 98.0%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC) to find that a generation ratio (isomer ratio) of the former compound to the latter compound was 98.9:1.1.

### (Example 9)

In a 300 ml four-necked flask equipped with a thermometer and a stirrer, 155.40 g of toluene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 22.20 g of toluene was dropped in 23 hours using a metering pump at an internal temperature of 5°C. After the completion of dropping, an aqueous layer and a toluene layer were separated. The obtained toluene layer was evaporated to dryness under reduced pressure to obtain 4.04 g (yield 99.0%) of a white crystal comprising ethyl 1-methyl-3-difluoromethylpyrazole-4-carboxylate and ethyl 1-methyl-5-difluoromethylpyrazole-4-carboxylate. The obtained white crystal was analyzed by high performance liquid chromatography (HPLC) to find that a generation ratio (isomer ratio) of the former compound to the latter compound was 99.0:1.0.

### (Example 10)

In a 50 ml four-necked flask equipped with a thermometer and a stirrer, 24.78 g of ethyl acetate and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 4.97 g of ethyl acetate was dropped in 16 hours using a metering pump at an internal temperature of 5°C. After the completion of dropping, an aqueous layer and an ethyl acetate layer were separated. The obtained ethyl acetate layer was evaporated to dryness under reduced pressure to obtain 3.91 g (yield 95.8%) of a light yellow crystal comprising ethyl 1-methyl-3-difluoromethylpyrazole-4-carboxylate and ethyl 1-methyl-5-difluoromethylpyrazole-4-carboxylate. The obtained light yellow crystal was analyzed by high performance liquid chromatography (HPLC) to find that a generation ratio (isomer ratio) of the former compound to the latter compound was 94.7:5.3.

### (Example 11)

In a 50 ml four-necked flask equipped with a thermometer and a stirrer, 24.8 g of o-xylene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 4.97 g of o-xylene was dropped in 22 hours using a metering pump at an internal temperature of 5°C. After the completion of dropping, an aqueous layer and an o-xylene layer were separated. The obtained o-xylene layer was evaporated to dryness under reduced pressure to obtain 3.59 g (yield 87.9%) of an orange-yellow crystal comprising ethyl 1-methyl-3-difluoromethylpyrazole-4-carboxylate and ethyl 1-methyl-5-difluoromethylpyrazole-4-carboxylate. The obtained orange-yellow crystal was analyzed by high performance liquid chromatography (HPLC) to find that a generation ratio (isomer ratio) of the former compound to the latter compound was 94.5:5.5.

### (Example 12)

In a 50 ml four-necked flask equipped with a thermometer and a stirrer, 8.60 g of toluene and 3.78 g (0.022 mol) of a 35% monoethylhydrazine aqueous solution were placed, and stirring was started. Into the resultant mixture, 13.1 g (0.02 mol) of a 34% toluene solution of ethyl 2-ethoxymethylene-4,4-difluoroacetoacetate was dropped in 4 hours using a metering pump at an internal temperature of 5°C. After the completion of dropping, an aqueous layer and a toluene layer were separated. The obtained toluene layer was evaporated to dryness under reduced pressure to obtain 4.15 g (yield 95.1%) of a white crystal comprising ethyl 1-ethyl-3-difluoromethylpyrazole-4-carboxylate and ethyl 1-ethyl-5-difluoromethylpyrazole-4-carboxylate. The obtained white crystal was analyzed by high performance liquid chromatography (HPLC) to find that a generation ratio (isomer ratio) of the former compound to the latter compound was 96.5:3.5.

### (Example 13)

In a 50 ml four-necked flask equipped with a thermometer and a stirrer, 8.60 g of toluene and 12.0 g (0.022 mol) of an 8.8% monomethylhydrazine aqueous solution were placed, and stirring was started. Into the resultant mixture, 14.1 g (0.02 mol) of a 34% toluene solution of ethyl 2-ethoxymethylene-4,4,4-trifluoroacetoacetate represented by the following formula (2-2) was dropped in 4 hours using a metering pump at an internal temperature of 5°C. After the completion of dropping, an aqueous layer and a toluene layer were separated. The obtained toluene layer was evaporated to dryness under reduced pressure to obtain 4.30 g (yield 96.8%) of a white crystal comprising ethyl 1-methyl-3-trifluoromethylpyrazole-4-carboxylate and ethyl 1-methyl-5-trifluoromethylpyrazole-4-carboxylate. The obtained white crystal was analyzed by high performance liquid chromatography (HPLC) to find that a generation ratio (isomer ratio) of the former compound to the latter compound was 96.1:3.9.

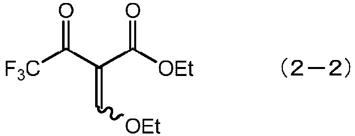

### (Example 14)

In a 50 ml four-necked flask equipped with a thermometer and a stirrer, 8.60 g of toluene and 3.78 g (0.022 mol) of a 35% monoethylhydrazine aqueous solution were placed, and stirring was started. Into the resultant mixture, 14.1 g (0.02 mol) of a 34% toluene solution of ethyl 2-ethoxymethylene-4,4,4-trifluoroacetoacetate was dropped in 4 hours using a metering pump at an internal temperature of 5°C. After the completion of dropping, an aqueous layer and a toluene layer were separated. The obtained toluene layer was evaporated to dryness under reduced pressure to obtain 4.50 g (yield 95.3%) of a white crystal comprising ethyl 1-ethyl-3-trifluoromethylpyrazole-4-carboxylate and ethyl 1-ethyl-5-trifluoromethylpyrazole-4-carboxylate. The obtained white crystal was analyzed by high performance liquid chromatography (HPLC) to find that a generation ratio (isomer ratio) of the former compound to the latter compound was 96.4:3.6.

### (Comparative Example 1)

In a 50 ml four-necked flask equipped with a thermometer and a stirrer, 24.8 g of toluene and 7.96 g (0.023 mol) of a 13.5% monomethylhydrazine aqueous solution were placed, and stirring was started. Into the resultant mixture, a mixed solution of 4.92 g (Net 4.44 g, 0.020 mol) of ethyl 2-ethoxymethylene-4,4-difluoroacetoacetate and 4.97 g of toluene was dropped in 5 minutes at an internal temperature of 5°C. After the completion of dropping, an aqueous layer and a toluene layer were separated. The obtained toluene layer was evaporated to dryness under reduced pressure to obtain 4.04 g (yield 81.9%) of a yellow-orange crystal comprising ethyl 1-methyl-3-difluoromethylpyrazole-4-carboxylate and ethyl 1-methyl-5-difluoromethylpyrazole-4-carboxylate. The obtained yellow-orange crystal was analyzed by high performance liquid chromatography (HPLC) to find that a generation ratio (isomer ratio) of the former compound to the latter compound was 83.7:16.3. In addition, the HPLC chart is shown in Figure 2.

### (Comparative Example 2)

In a 50 ml four-necked flask equipped with a thermometer and a stirrer, 2.90 g (0.022 mol) of a 35% monomethylhydrazine aqueous solution was placed, and stirring was started. Into the solution, 4.45 g of ethyl 2-ethoxymethylene-4,4-difluoroacetoacetate was dropped in 5 minutes at an internal temperature of 5°C. After the completion of dropping, an aqueous layer was separated and removed to obtain 3.6 g (yield 45%) of reddish brown oil containing ethyl 1-methyl-3-difluoromethylpyrazole-4-carboxylate and ethyl 1-methyl-5-difluoromethylpyrazole-4-carboxylate. The obtained reddish brown oil was analyzed by high performance liquid chromatography (HPLC) to find that a generation ratio (isomer ratio) of the former compound to the latter compound was 77.8:22.2.

### (Comparative Example 3)

In a 50 ml four-necked flask equipped with a thermometer and a stirrer, 0.89 g of toluene and 15.92 g (0.046 mol) of a 13.5% monomethylhydrazine aqueous solution were placed, and stirring was started. Into the resultant mixture, a mixed solution of 9.84 g (Net 8.88 g, 0.040 mol) of ethyl 2-ethoxymethylene-4,4-difluoroacetoacetate and 0.89 g of toluene was dropped in 1 hour using a metering pump at an internal temperature of 5°C. In addition, a cream colored crystal had been precipitated in the reaction liquid when the dropping was completed. When the temperature of the reaction liquid was returned to room temperature after the completion of dropping, the crystal was dissolved to be an emulsion. The emulsion was extracted by adding 10 g of toluene to the reaction liquid, and the obtained toluene layer was evaporated to dryness under reduced pressure to obtain 7.73 g (yield 65.7%) of an orange-yellow crystal comprising ethyl 1-methyl-3-difluoromethylpyrazole-4-carboxylate and ethyl 1-methyl-5-difluoromethylpyrazole-4-carboxylate. The obtained orange-yellow crystal was analyzed by high performance liquid chromatography (HPLC) to find that a generation ratio (isomer ratio) of the former compound to the latter compound was 89.0:11.0.

### (Comparative Example 4)

In a 50 ml four-necked flask equipped with a thermometer and a stirrer, 0.89 g of toluene and 15.92 g (0.046 mol) of a 13.5% monomethylhydrazine aqueous solution were placed, and stirring was started. Into the resultant mixture, a mixed solution of 9.84 g (Net 8.88 g, 0.040 mol) of ethyl 2-ethoxymethylene-4,4-difluoroacetoacetate and 0.89 g of toluene was dropped in 24 hours using a metering pump at an internal temperature of 5°C. In addition, a cream colored crystal had been precipitated in the reaction liquid when the dropping was completed. When the temperature of the reaction liquid was returned to room temperature after the completion of dropping, the crystal was dissolved to be an emulsion. The emulsion was extracted by adding 10 g of toluene to the reaction liquid, and the obtained toluene layer was evaporated to dryness under reduced pressure to obtain 6.98 g (yield 70.4%) of an orange-yellow crystal comprising ethyl 1-methyl-3-difluoromethylpyrazole-4-carboxylate and ethyl 1-methyl-5-difluoromethylpyrazole-4-carboxylate. The obtained orange-yellow crystal was analyzed by high performance liquid chromatography (HPLC) to find that a generation ratio (isomer ratio) of the former compound to the latter compound was 85.0:15.0.

### (Comparative Example 5)

In a 100 ml four-necked flask equipped with a thermometer and a stirrer, 8.67 g of water, 1.68 g of a 48% sodium hydroxide aqueous solution, and 4.08 g (0.031 mol) of a 35% monomethylhydrazine aqueous solution were placed, and stirring was started. Into the resultant mixture, a mixed solution of 4.44 g (0.020 mol) of ethyl 2-ethoxymethylene-4,4-difluoroacetoacetate and 38.5 g of toluene was dropped in 5 minutes at an internal temperature of 50°C. After the completion of dropping, the reaction liquid was stirred at an internal temperature of 50°C for 10 minutes. Next, an aqueous layer and a toluene layer were separated, and the obtained toluene layer was evaporated to dryness under reduced pressure to obtain 2.94 g of a yellow-white crystal comprising ethyl 1-methyl-3-difluoromethylpyrazole-4-carboxylate and ethyl 1-methyl-5-difluoromethylpyrazole-4-carboxylate. The obtained yellow-white crystal was analyzed by high performance liquid chromatography (HPLC), and quantitative analysis was conducted by an absolute calibration curve method to find that a yield was 72.0% and a generation ratio (isomer ratio) of the former compound to the latter compound was 99.1:0.9. In addition, the aqueous layer obtained by separation was analyzed by HPLC to find that 1-methyl-3-difluoromethylpyrazole-4-carboxylic acid being a hydrolysis product of ethyl 1-methyl-3-difluoromethylpyrazole-4-carboxylate was contained with a yield of 23.7%.

The reaction conditions and so on, yields, and isomer ratios of the above-described Examples 1 to 14 and Comparative Examples 1 to 5 are shown together in Tables 1 and 2.

**Table 1**

| | First reaction liquid | | | | Second reaction liquid | | | | | | | First organic solvent + Second organic solvent (g) |
|---|---|---|---|---|---|---|---|---|---|---|---|---|
| | Alkyl hydrazine | | First organic solvent | | Acyl acetic acid ester derivative | | | | | Second organic solvent | | |
| | R₁ | Quantity (g) | Kind | Quantity (g) | R₂ | R₃ | R₄ | R₅ | Quantity (g) | Kind | Quantity (g) | |
| Example 1 | Me | 2.15 | Toluene | 49.55 | F | H | Et | Et | 8.88 | Toluene | 9.95 | 59.5 |
| Example 2 | Me | 1.05 | Toluene | 4.45 | F | H | Et | Et | 4.45 | Toluene | 4.45 | 8.9 |
| Example 3 | Me | 1.05 | Toluene | 8.60 | F | H | Et | Et | 4.45 | Toluene | 8.65 | 17.3 |
| Example 4 | Me | 1.05 | Toluene | 18.00 | F | H | Et | Et | 4.45 | Toluene | 8.65 | 26.7 |
| Example 5 | Me | 1.07 | Toluene | 24.80 | F | H | Et | Et | 4.44 | Toluene | 4.92 | 29.7 |
| Example 6 | Me | 1.07 | Toluene | 24.80 | F | H | Et | Et | 4.44 | Toluene | 4.97 | 29.8 |
| Example 7 | Me | 1.07 | Toluene | 24.80 | F | H | Et | Et | 4.44 | Toluene | 4.97 | 29.8 |
| Example 8 | Me | 1.07 | Toluene | 79.92 | F | H | Et | Et | 4.44 | Toluene | 8.88 | 88.8 |
| Example 9 | Me | 1.07 | Toluene | 155.40 | F | H | Et | Et | 4.44 | Toluene | 22.20 | 177.6 |
| Example 10 | Me | 1.07 | Ethyl acetate | 24.78 | F | H | Et | Et | 4.44 | Ethyl acetate | 4.97 | 29.8 |
| Example 11 | Me | 1.07 | o-Xylene | 24.80 | F | H | Et | Et | 4.44 | o-Xylene | 4.97 | 29.8 |
| Example 12 | Et | 1.32 | Toluene | 8.60 | F | H | Et | Et | 4.45 | Toluene | 8.65 | 17.3 |
| Example 13 | Me | 1.05 | Toluene | 8.60 | F | F | Et | Et | 4.79 | Toluene | 9.31 | 17.9 |
| Example 14 | Et | 1.32 | Toluene | 8.60 | F | F | Et | Et | 4.79 | Toluene | 9.31 | 17.9 |
| Comparative Example 1 | Me | 1.07 | Toluene | 24.80 | F | H | Et | Et | 4.44 | Toluene | 4.92 | 29.7 |
| Comparative Example 2 | Me | 1.01 | - | - | F | H | Et | Et | 4.45 | - | - | - |
| Comparative Example 3 | Me | 2.15 | Toluene | 0.89 | F | H | Et | Et | 8.88 | Toluene | 0.89 | 1.8 |
| Comparative Example 4 | Me | 2.15 | Toluene | 0.89 | F | H | Et | Et | 8.88 | Toluene | 0.89 | 1.8 |
| Comparative Example 5 * | Me | 1.43 | - | - | F | H | Et | Et | 4.44 | Toluene | 38.50 | 38.5 |

| | | | | | | | | | | | | |
|---|---|---|---|---|---|---|---|---|---|---|---|---|
| *1: Reaction was conducted in the presence of NaOH | | | | | | | | | | | | |

**Table 2**

| | (First organic solvent + second organic solvent)/acyl acetic acid ester derivative | First organic solvent/ (first organic solvent + second organic solvent) | Dropping time | Temperature | Yield | Isomer ratio (%) | |
|---|---|---|---|---|---|---|---|
| | (times) | (%) | (h) | (°C) | (%) | *1 | *2 |
| Example 1 | 6.7 | 83.3 | 16 | 5 | 92.8 | 97.4 | 2.6 |
| Example 2 | 2.0 | 50.0 | 4 | 5 | 95.5 | 94.1 | 5.9 |
| Example 3 | 3.9 | 49.9 | 4 | 5 | 96.7 | 96.9 | 3.1 |
| Example 4 | 6.0 | 67.5 | 24 | 5 | 98.0 | 98.9 | 1.1 |
| Example 5 | 6.7 | 83.5 | 0.5 | 5 | 90.0 | 91.5 | 8.5 |
| Example 6 | 6.7 | 83.2 | 22 | 50 | 94.5 | 94.2 | 5.8 |
| Example 7 | 6.7 | 83.2 | 1 | 5 | 94.5 | 97.6 | 2.4 |
| Example 8 | 20.0 | 90.0 | 18 | 5 | 98.0 | 98.9 | 1.1 |
| Example 9 | 40.0 | 87.5 | 23 | 5 | 99.0 | 99.0 | 1.0 |
| Example 10 | 6.7 | 83.4 | 16 | 5 | 95.8 | 94.7 | 5.3 |
| Example 11 | 6.7 | 83.5 | 22 | 5 | 87.9 | 94.5 | 5.5 |
| Example 12 | 3.9 | 49.9 | 4 | 5 | 95.1 | 96.5 | 3.5 |
| Example 13 | 3.7 | 48.0 | 4 | 5 | 96.8 | 96.1 | 3.9 |
| Example 14 | 3.7 | 48.0 | 4 | 5 | 95.3 | 96.4 | 3.6 |
| Comparative Example 1 | 6.7 | 83.5 | 0.08 | 5 | 81.9 | 83.7 | 16.3 |
| Comparative Example 2 | - | - | 0.08 | 5 | 45.0 | 77.8 | 22.2 |
| Comparative Example 3 | 0.2 | 50.0 | 1 | 5 | 65.7 | 89.0 | 11.0 |
| Comparative Example 4 | 0.2 | 50.0 | 24 | 5 | 70.4 | 85.0 | 15.0 |
| Comparative Example 5 | 8.7 | - | 0.08 | 50 | 72.0 | 99.1 | 0.9 |

| | | | | | | | |
|---|---|---|---|---|---|---|---|
| *1: 1-Substituted-3-fluoroalkylpyrazole-4-carboxylic acid ester *2: 1-Substituted-5-fluoroalkylpyrazole-4-carboxylic acid ester | | | | | | | |

### Industrial Applicability

The production method of the present invention is suitable as a method for industrially producing a 1-substituted-3-fluoroalkylpyrazole-4-carboxylic acid ester that is useful as a synthetic intermediate or the like for medical and pharmaceutical products and agricultural chemicals.

## Claims

1. A method for producing a 1-substituted-3-fluoroalkylpyrazole-4-carboxylic acid ester represented by the following general formula (3), the method comprising:
a step of adding, to a first reaction liquid containing an alkyl hydrazine represented by the following general formula (1) and a first organic solvent, a second reaction liquid containing an acyl acetic acid ester derivative represented by the following general formula (2) and a second organic solvent in 0.5 to 30 hours to react the first reaction liquid with the second reaction liquid at a reaction temperature of -5 to 80°C under stirring in the absence of a base and an acid,
wherein the first organic solvent and the second organic solvent are each at least any one of benzene, toluene, xylene, chlorobenzene, dichlorobenzene, ethyl acetate, butyl acetate, and dimethyl carbonate,
a total mass of the first organic solvent and the second organic solvent is 1 to 60 times a mass of the acyl acetic acid ester derivative, and
an amount of the first organic solvent in a total amount of the first organic solvent and the second organic solvent is 40 to 95% by mass.
**R₁-NHNH₂** **(1)**
(in the general formula (1), R₁ represents a C1-C6 alkyl group which may be substituted) 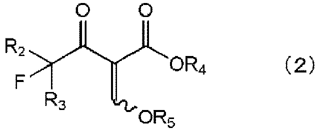 (in the general formula (2), R₂ represents a hydrogen atom or a halogen atom, R₃ represents a hydrogen atom, a fluorine atom, or a C1-C12 alkyl group which may be substituted with a chlorine atom or a fluorine atom, and R₄ and R₅ each independently represent a C1-C6 alkyl group) 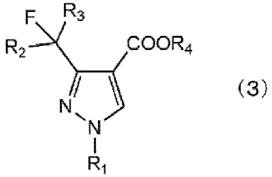 (in the general formula (3), R₁ represents a C1-C6 alkyl group which may be substituted, R₂ represents a hydrogen atom or a halogen atom, R₃ represents a hydrogen atom, a fluorine atom, or a C1-C12 alkyl group which may be substituted with a chlorine atom or a fluorine atom, and R₄ represents a C1-C6 alkyl group)

2. The method for producing a 1-substituted-3-fluoroalkylpyrazole-4-carboxylic acid ester according to Claim 1, wherein the amount of the first organic solvent in the total amount of the first organic solvent and the second organic solvent is 65 to 92% by mass.

3. The method for producing a 1-substituted-3-fluoroalkylpyrazole-4-carboxylic acid ester according to Claim 1 or 2, wherein the first organic solvent and the second organic solvent are each at least any one of toluene, xylene, and ethyl acetate.

4. The method for producing a 1-substituted-3-fluoroalkylpyrazole-4-carboxylic acid ester according to any one of Claims 1 to 3, wherein the total mass of the first organic solvent and the second organic solvent is 5 to 60 times the mass of the acyl acetic acid ester derivative.

5. The method for producing a 1-substituted-3-fluoroalkylpyrazole-4-carboxylic acid ester according to any one of Claims 1 to 4, wherein the amount of the acyl acetic acid ester derivative contained in the second reaction liquid is 0.8 to 1.2 molar equivalents relative to the amount of the alkyl hydrazine.

## Patentansprüche

1. Verfahren zur Herstellung eines 1 -substutierten-3-Fluoralkylpyrazol-4-carbonsäureesters der folgenden allgemeinen Formel (3), wobei das Verfahren folgendes umfasst:
einen Schritt des Zugebens, zu einer ersten Reaktionsflüssigkeit, die ein Alkylhydrazin der folgenden allgemeinen Formel (1) und ein erstes organisches Lösungsmittel enthält, einer zweiten Reaktionsflüssigkeit, die ein Acylessigsäureester-Derivat der folgenden allgemeinen Formel (2) und ein zweites organisches Lösungsmittel enthält, in 0,5 bis 30 Stunden, um die erste Reaktionsflüssigkeit mit der zweiten Reaktionsflüssigkeit bei einer Reaktionstemperatur von -5 bis 80°C unter Rühren in Abwesenheit einer Base und einer Säure umzusetzen, wobei
das erste organische Lösungsmittel und das zweite organische Lösungsmittel jeweils wenigstens eines von Benzol, Toluol, Xylol, Chlorbenzol, Dichlorbenzol, Ethylacetat, Butylacetat und Dimethylcarbonat sind,
die Gesamtmasse des ersten organischen Lösungsmittels und des zweiten organischen Lösungsmittels das 1- bis 60-fache der Masse des Acylessigsäureester-Derivats beträgt, und die Menge des ersten organischen Lösungsmittels in der Gesamtmenge des ersten organischen Lösungsmittels und des zweiten organischen Lösungsmittels 40 bis 95 Masseprozent beträgt:
R₁-NHNH₂ (1)
(in der allgemeinen Formel (1) steht R₁ für eine C1-C6 Alkylgruppe, die substituiert sein kann), 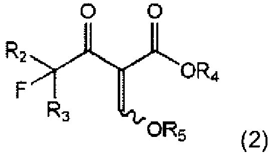 (in der allgemeinen Formel (2) steht R₂ für ein Wasserstoffatom oder ein Halogenatom, R₃ steht für ein Wasserstoffatom, ein Fluoratom oder eine C1-C12 Alkylgruppe, die mit einem Chloratom oder einem Fluoratom substituiert sein kann, und R₄ und R₅ stehen jeweils unabhängig für eine C1-C6 Alkylgruppe), 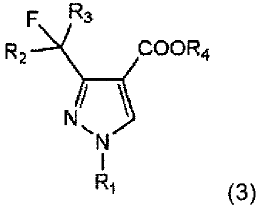 (in der allgemeinen Formel (3) steht R₁ für eine C1-C6 Alkylgruppe, die substituiert sein kann, R₂ steht für ein Wasserstoffatom oder ein Halogenatom, R₃ steht für ein Wasserstoffatom, ein Fluoratom oder eine C1-C12 Alkylgruppe, die mit einem Chloratom oder einem Fluoratom substituiert sein kann, und R₄ steht für eine C1-C6 Alkylgruppe).

2. Das Verfahren zur Herstellung eines 1-substutierten-3-Fluoralkylpyrazol-4-carbonsäureesters nach Anspruch 1, wobei die Menge des ersten organischen Lösungsmittels in der Gesamtmenge des ersten organischen Lösungsmittels und des zweiten organischen Lösungsmittels 65 bis 92 Masseprozent beträgt

3. Das Verfahren zur Herstellung eines 1-substutierten-3-Fluoralkylpyrazol-4-carbonsäureesters nach Anspruch 1 oder 2, wobei das erste organische Lösungsmittel und das zweite organische Lösungsmittel jeweils wenigstens eines von Toluol, Xylol und Ethylacetat sind.

4. Das Verfahren zur Herstellung eines 1-substutierten-3-Fluoralkylpyrazol-4-carbonsäureesters nach einem der Ansprüche 1 bis 3, wobei die Gesamtmasse des ersten organischen Lösungsmittels und des zweiten organischen Lösungsmittels das 5- bis 60-fache der Masse des Acylessigsäureester-Derivats beträgt.

5. Das Verfahren zur Herstellung eines 1-substutierten-3-Fluoralkylpyrazol-4-carbonsäureesters nach einem der Ansprüche 1 bis 4, wobei die Menge des Acylessigsäureester-Derivats, das in der zweiten Reaktionsflüssigkeit enthalten ist, 0,8 bis 1,2 molare Äquivalente, bezogen auf die Menge des Alkylhydrazins, beträgt.

## Revendications

1. Procédé pour produire un ester d'acide 3-fluoroalkylpyrazole-4-carboxylique 1-substitué représenté par la formule générale (3) suivante, le procédé comprenant :
une étape d'addition, à un premier liquide réactionnel contenant une alkylhydrazine représentée par la formule générale (1) suivante et un premier solvant organique, d'un deuxième liquide réactionnel contenant un dérivé ester d'acide acylacétique représenté par la formule générale (2) suivante et un deuxième solvant organique, en 0,5 à 30 heures, pour que le premier liquide réactionnel réagisse avec le deuxième liquide réactionnel à une température réactionnelle de -5 à 80°C sous agitation en l'absence d'une base et d'un acide,
dans lequel chacun parmi le premier solvant organique et le deuxième solvant organique est au moins l'un quelconque parmi le benzène, le toluène, le xylène, le chlorobenzène, le dichlorobenzène, l'acétate d'éthyle, l'acétate de butyle, et le carbonate de diméthyle,
la masse totale du premier solvant organique et du deuxième solvant organique est de 1 à 60 fois la masse du dérivé ester d'acide acylacétique, et
la quantité du premier solvant organique par rapport à la quantité totale du premier solvant organique et du deuxième solvant organique est de 40 à 95 % en masse :
R₁-NHNH₂ (1)
(dans la formule générale (1), R₁ représente un groupe alkyle en C₁ à C₆ qui peut être substitué) 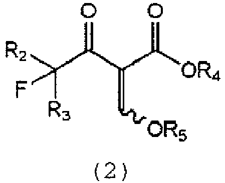 (dans la formule générale (2), R₂ représente un atome d'hydrogène ou un atome d'halogène, R₃ représente un atome d'hydrogène, un atome de fluor, ou un groupe alkyle en C₁ à C₁₂ qui peut être substitué par un atome de chlore ou un atome de fluor, et chacun de R₄ et R₅ représente indépendamment un groupe alkyle en C₁ à C₆) 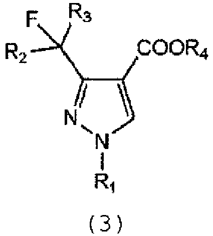 (dans la formule générale (3), R₁ représente un groupe alkyle en C₁ à C₆ qui peut être substitué, R₂ représente un atome d'hydrogène ou un atome d'halogène, R₃ représente un atome d'hydrogène, un atome de fluor, ou un groupe alkyle en C₁ à C₁₂ qui peut être substitué par un atome de chlore ou un atome de fluor, et R₄ représente un groupe alkyl en C₁ à C₆).

2. Procédé pour produire un ester d'acide 3-fluoroalkylpyrazole-4-carboxylique 1-substitué selon la revendication 1, dans lequel la quantité du premier solvant organique par rapport à la quantité totale du premier solvant organique et du deuxième solvant organique est de 65 à 92 % en masse.

3. Procédé pour produire un ester d'acide 3-fluoroalkylpyrazole-4-carboxylique 2-substitué selon la revendication 1 ou 2, dans lequel chacun parmi le premier solvant organique et le deuxième solvant organique est au moins l'un quelconque parmi le toluène, le xylène, et l'acétate d'éthyle.

4. Procédé pour produire un ester d'acide 3-fluoroalkylpyrazole-4-carboxylique 1-substitué selon l'une quelconque des revendications 1 à 3, dans lequel la masse totale du premier solvant organique et du deuxième solvant organique est de 5 à 60 fois la masse du dérivé ester d'acide acylacétique.

5. Procédé pour produire un ester d'acide 3-fluoroalkylpyrazole-4-carboxylique 1-substitué selon l'une quelconque des revendications 1 à 4, dans lequel la quantité du dérivé ester d'acide acylacétique contenu dans le deuxième liquide réactionnel est de 0,8 à 1,2 équivalents molaires par rapport à la quantité de l'alkylhydrazine.
